# EUROPEAN PATENT SPECIFICATION

(11) **EP 2 480 179 B1**
(45) Date of publication and mention of the grant of the patent: **17.08.2016**
(21) Application number: 10759766.8
(22) Date of filing: 09.09.2010
(51) Int. Cl.: A61F 2/90

(54) **INTEGRATED STENT RETRIEVAL LOOP ADAPTED FOR SNARE REMOVAL AND/OR OPTIMIZED PURSE STRINGING**
INTEGRIERTE STENTERFASSUNGSSCHLEIFE ZUR SCHLINGENENTNAHME UND/ODER FÜR OPTIMIERTE RAFFNÄHUNG
BOUCLE DE RÉCUPÉRATION D'ENDOPROTHÈSE INTÉGRÉE POUR ENLÈVEMENT À L'ANSE ET/OU CORDAGE DE BOURSE OPTIMISÉ

(30) Priority: 21.09.2009 US 244206 P
(43) Date of publication of application: 01.08.2012
(60) Divisional application: 16167974.1
(73) Proprietor: Boston Scientific Scimed, Inc., Maple Grove, MN 55311 (US)
(72) Inventor: WOOD, Mark, Shrewsbury MA 01545 (US); AMOS, Devon, Boston MA 02118 (US); NORTON, Paul, K., Lunenburg MA 01462 (US)
(74) Representative: Vossius & Partner Patentanwälte Rechtsanwälte mbB
(86) International application number: PCT/US2010/048191
(87) International publication number: WO 2011/034768

(56) References cited:
- EP-A2- 1 518 518
- WO-A1-02/083037
- WO-A1-2006/081448
- WO-A2-2006/124541
- US-A1- 2002 143 387

## Description

### FIELD OF THE INVENTION:

The present invention relates to implantable stents having a stent retrieval member or loop for easy retrieval and/or repositioning of the implanted stent.

### BACKGROUND OF THE INVENTION:

An intraluminal prosthesis is a medical device used in the treatment of diseased bodily lumens. One type of intraluminal prosthesis used in the repair and/or treatment of diseases in various body vessels is a stent. A stent is a generally longitudinal tubular device formed of biocompatible material which is useful to open and support various lumens in the body. For example, stents may be used in the vascular system, urogenital tract, esophageal tract, tracheal/bronchial tubes and bile duct, as well as in a variety of other applications in the body. These devices are implanted within the vessel to open and/or reinforce collapsing or partially occluded sections of the lumen.

Stents generally include an open flexible configuration. This configuration allows the stent to be inserted through curved vessels. Furthermore, this configuration allows the stent to be configured in a radially compressed state for intraluminal catheter implantation. Once properly positioned adjacent the damaged vessel, the stent is radially expanded so as to support and reinforce the vessel. Radial expansion of the stent may be accomplished by inflation of a balloon attached to the catheter or the stent may be of the self-expanding variety which will radially expand once deployed.

Prior retrieval systems, for example as described in U.S. Pat No. 6,821,291 to Bolea et al., may appear easy to use, but often are limited to a specific tool for removal and/or require certain user-sensitive techniques, such as twisting or turning in order to reposition or remove the stent. Moreover, in smaller stents, such as biliary stents, the spacing between conventional stent segments is generally smaller than the size of standard forceps or graspers, making it even difficult to grab a portion of the stent.

WO 2006/124541 discloses a braided stent having an integral retrieval and/or repositioning loop includes a plurality of wires having first and second ends interbraided in a braided pattern to form a tubular stent having opposed atraumatic first and second open ends with each open end having a circumference; wherein said first and second wires ends are disposed at said second stent open end and said wires are looped at said second stent open end so that none of the first or second wires ends are exposed at the circumference of second stent open end; wherein at least of two of said wires are formed into a repositioning and/or retrieval loop having an elongated portion circumferentially disposed at said first opposed open end; and wherein said reposition and/or retrieval loop comprises two sections which run adjacent to each other prior to crossing to permit grabbing of both sections simultaneously by a -practitioner.

### SUMMARY OF THE INVENTION

The present invention provides an implantable device, as disclosed in the appended claims, for example a stent, including a braided stent, having a retrieval and/or repositioning member. The implantable device is formed from one or more elongated filaments wound or braided to form a tubular device having opposed first open end, a second open end, a tubular body therebetween. The device has an interior surface and an exterior surface. The retrieval and/or repositioning member includes an elongated portion extending from the first open end and the retrieval and/or repositioning member interlooping circumferentially about the first open end such that force exerted on the elongated portion causes radially contraction of the device.

In another aspect of the present invention, one or more elongated filaments are wound or braided to form a tubular implantable device or stent having a retrieval and/or repositioning member and opposed first open end and a second open end with each open end having a circumference and a tubular body therebetween is provided. The first open end is defined by series of closed-end loops. The retrieval and/or repositioning member has a first section including at least one elongated closed-end loop extending from the first open end, and a second section emerging from the braided tubular body, interwoven with at least one closed-end loop and integrally extending into the first section whereby force exerted on the elongated closed-end loop causes radially contraction of the tubular device.

In an example not forming part of the invention as claimed, a method for producing a tubular wound or braided implantable device or stent having opposed first end and second end and having an integral retrieval and/or repositioning loop at the first end is provided. The tubular wound or braided device or stent includes the steps of selecting a single elongate biocompatible filament having opposed ends; forming a retrieval and/or repositioning member from the single filament comprising an elongated loop which extends above the first end to permit grabbing of the loop by a practitioner to radially contract the stent; and winding or braiding the single filament, optionally with other filaments, to form the device or stent.

### BRIEF DESCRIPTION OF THE DRAWINGS:

FIG. 1 is a partial expanded view of a stent of the prior art.
FIG. 2 is another view of the prior art stent of FIG. 1 being pulled by a retrieval device.
FIG. 3 depicts a braided stent with closed-end loop design having a retrieval and/or repositioning member.
FIG. 4 is a perspective view of one end of the stent of FIG. 3 having a retrieval and/or repositioning member.
FIG. 5 is an expanded view of the retrieval and/or repositioning member.
FIG. 6 is an expanded view of the retrieval and/or repositioning member of the present invention being pulled by a retrieval device.
FIG. 7 is another view of the retrieval and/or repositioning member of FIG. 6 in a retracted or compressed state.
FIG. 8 is an expanded view of the stent end of the present invention including a retrieval and/or repositioning member having an outwardly directed bent portion.
FIG. 9 is a side view of the stent end of FIG. 8.
FIG. 10 is an expanded view of the stent end of the present invention including a retrieval and/or repositioning member having an inwardly directed bent portion.
FIG. 11 is an expanded view of the stent end of the present invention including a retrieval and/or repositioning member having an outwardly directed twisted portion.
FIG. 12 is an expanded view of the stent end of the present invention including a retrieval and/or repositioning member having an inwardly directed twisted portion.

### DETAILED DESCRIPTION:

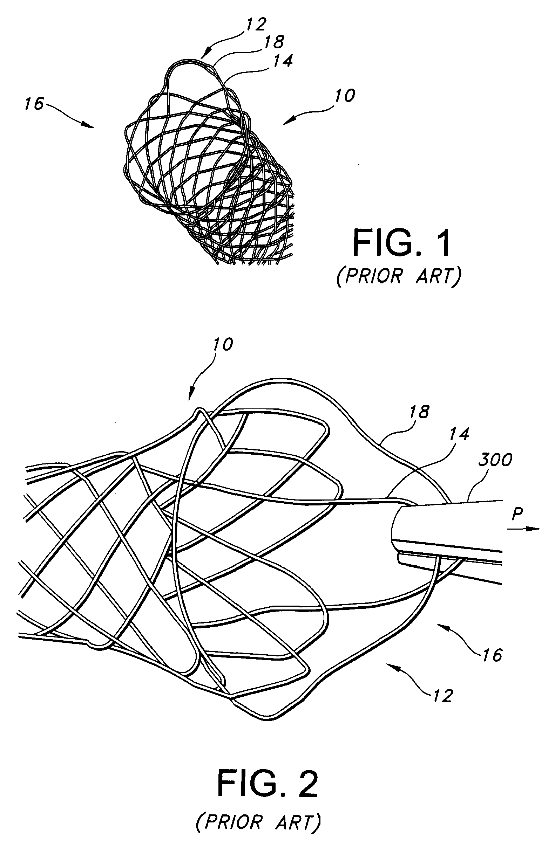

FIG. 1 depicts a prior art stent **10** including a retrieval and/or repositioning loop **12.** The retrieval and/or repositioning loop **12** includes two wires **14, 18** that are circumferentially disposed about the end **16** of stent **10.** The two wires **14, 18** extend outwardly from the stent end **16** to permit access to the stent **10** by a practitioner with a retrieval device such as rat tooth forceps or hooking device. The two wires **14, 18** cooperatively work in conjunction with each other to cinch the end **16** of the stent **10** and radially contract the stent **10** body when pulled on by a retrieval device. Specifically, when the two wires **14, 18** of the retrieval and/or repositioning loop **12** are accessed and pulled, the two wires **14, 18** slide by each other to allow the stent end **16** to cinch. Once the stent end **16** is cinched, then continued pulling on the retrieval and/or repositioning loop **12** axially compresses or radially contracts the body of the stent **10** from the cinched stent end down to the other end. In use, however, both wires **14, 18** are pulled together to provide for radial contraction of the stent **10.** Further, these wires **14, 18** slide about each other when being pulled to permit radial contraction of the stent **10.** The retrieval and/or repositioning loop **12** of the prior art may use a retrieval device such as rat tooth forceps or hook to engage its retrieval and/or repositioning loop because the other devices may pinch the two wires **14, 18** and thereby hindering the two wires **14, 18** from sliding by each other. Other useful retrieval devices include but are not limited to needle nose pliers, radial jaws or a snare. Further details of this prior art stent **10** with its retrieval and/or repositioning loop **12** may be found in U.S. Patent Application Publication No. 2006/0276887 to Brady et al., the contents of which are incorporated herein by reference. Moreover, a retrieval and/or repositioning loop which is not integrally from the wires braided to form a stent is disclosed in U.S. Patent Application Publication No. 2006/0190075 to Jordan et al.

FIG. 2 shows the prior art stent **10** being pulled **(P)** by a retrieval device **300** in the direction away from the stent end **16.** The retrieval device **300** hold both wires **14, 18** in a fixed position as it pulls the stent **10.** The retrieval device **300** hinders the wires **14, 18** from moving about each other which causes the stent end **16** to remain in a flared state and prevent the wires **14, 18** from fully cinching the stent end **16.** Additionally, FIG. 2 shows that the stent **10** body is not fully radially contracted due to the reduced ability of the wires **14, 18** to slide about each other.

Thus, there is a need for a single wire retrieval and/or repositioning member that provides both improved stent end cinching and improved stent body radial contraction. Further, there is a need for a single wire retrieval and/or repositioning member that is capable of cinching the end of the stent and radially contracting the stent body using a variety of devices used by a practitioner. Furthermore, there is a need for a single wire retrieval and/or repositioning member that provides for substantially even radial contraction of the stent end and stent body and permits easy access by a practitioner to the pulling member of the stent.

The present invention provides at least one single wire retrieval and/or repositioning member which is integral and formed from one of the filaments or wires used to form the braided stent. The retrieval and/or repositioning member is designed to provide a structure which has the required tensile strength to prevent fracture or damage to the stent when force is applied to reposition or retrieve the stent, yet allows for a very low delivery profile such that it can easily be loaded onto a delivery device without interfering with the deployment into the body or requiring increased deployment force. Because the retrieval and/or repositioning member is an integral part of the actual braiding or winding for the stent structure *per se,* as opposed to being a separate add-on element, no joining, i.e., welding, crimping, twisting, of the retrieval and/or repositioning member to the stent structure is necessary. Tensile strength of the retrieval and/or repositioning member may thus be maximized while concomitantly maintaining the lowest profile for delivery to a patient. The wire or wires used to form at least one retrieval and/or repositioning member may be of the same type and material as the other wires forming the braided stent, or alternatively they may be made from different types or materials. In one desirable embodiment, the retrieval and/or repositioning member is made from a single wire which is also used to form the braided stent or at least part of the braided stent. In this manner, the retrieval and/or repositioning member can further seamlessly transition into the body of the stent. As used herein, the phrase "retrieval and/or repositioning member" refers to a retrieval member, a repositioning member, or a combination thereof which is integrally formed with a stent and, when a longitudinally pulling force is applied thereto, aids in the radial contraction or cinching of the entire stent equally to facilitate movement, retrieval and/or repositioning of the stent.

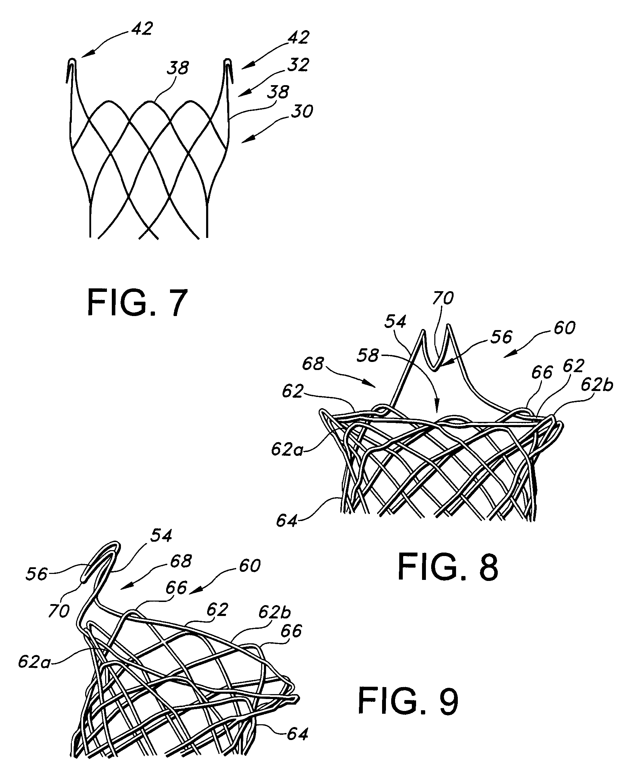

More than one retrieval and/or repositioning member may be incorporated into the stent. For example, each stent end might have one or more retrieval and/or repositioning members, as shown in FIG. 7. In some embodiments only one retrieval and/or repositioning member is present at one or more ends.

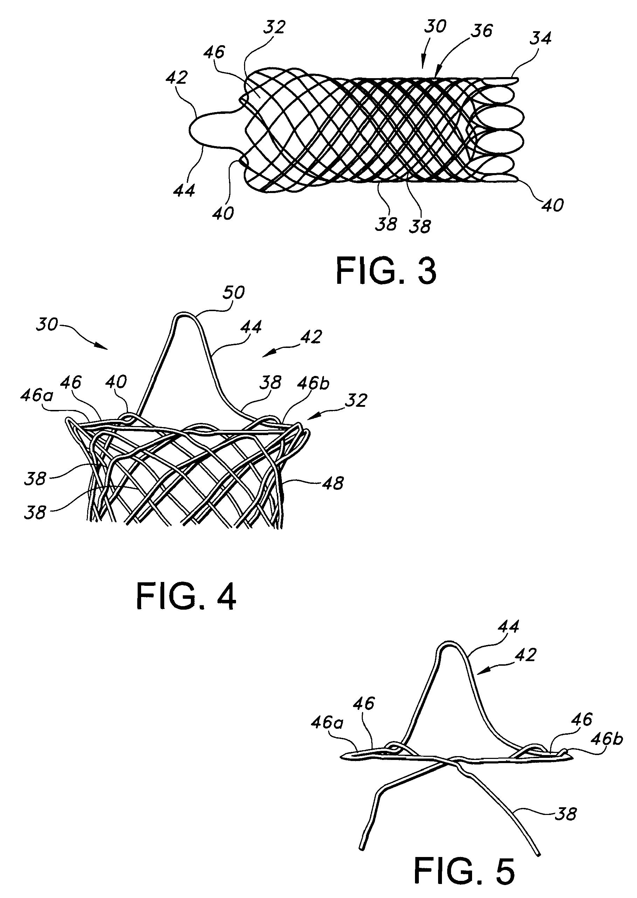

FIG. 3 depicts stent **30**. Stent **30** is a hollow tubular structure having opposed first and second open ends **32, 34** and having a tubular wall **36** therebetween. The tubular wall **36** has a plurality of elongate wires **38** formed into the tubular wall **36.** The elongate wires **38** traverse the length of the stent **30** in a direction traverse to the longitudinal length of the stent **30.** The elongate wires **38** may be formed into the tubular wall **36** by braiding the wires **38,** winding the wires **38** or even winding a single wire **38,** knitting the wires **38,** and combinations thereof. Preferably, the wires **38** are braided in a braided pattern to form the tubular wall **36.** A useful nonlimiting braided pattern includes a one over and one under pattern, but other patterns may suitably be used.

As depicted in FIG. 3, stent **30** is desirably an atraumatic stent having close-loop ends **40** defining the circumference of the opposed first and second open ends **32, 34.** The elongate wire **38** terminating at open end **32** are looped over to form a closed-end loop **40** and reintroduced into the stent to form the completed braided sent. After the braided stent is formed, the ends of the wire may be welded or otherwise connected together forming a braided stent with no open ends, open loops or sharp edges. Additionally, multiple wires **38** may be used in forming the stent and the wires **38** are mated to form closed-loops and adjacently mated wires are secured to one and the other by mechanical means, such as welds.

The stent **30** is desirably an atraumatic stent having no sharp terminating members at one or both of the opposed first and second open ends **32, 34.** The elongate wires **38** terminating at open end **32** are mated to form closed-end loops **40** and adjacently mated wires are secured to one and the other by mechanical means, such as welds. The positioning of adjacently mated wires to form closed-end loop designs is further described in U.S. Published Application Nos. US 2005-0049682 A1, and 2006-0116752 A1. Desirably, the elongate wires **38** terminating at open end **32** are in a cathedral type arch or loop configuration. Further details of the cathedral type of arch or closed-loop configuration may be found in U.S. Application Publication No. 2005-0256563 A1.

In any event, the current invention is useful with various stent designs, including those without atraumatic ends.

Desirably, the wires **38** are made from any suitable implantable material, including without limitation nitinol, stainless steel, cobalt-based alloy such as Elgiloy®, platinum, gold, titanium, tantalum, niobium, polymeric materials and combinations thereof. Useful and nonlimiting examples of polymeric stent materials include poly(L-lactide) (PLLA), poly(D,L-lactide) (PLA), poly(glycolide) (PGA), poly(L-lactide-co-D,L-lactide) (PLLA/PLA), poly(L-lactide-co-glycolide) (PLLA/PGA), poly(D,L-lactide-co-glycolide) (PLA/PGA), poly(glycolide-co-trimethylene carbonate) (PGA/PTMC), polydioxanone (PDS), Polycaprolactone (PCL), polyhydroxybutyrate (PHBT), poly(phosphazene) poly(D,L-lactide-co-caprolactone) PLA/PCL), poly(glycolide-co-caprolactone) (PGA/PCL), poly(phosphate ester) and the like. Wires made from polymeric materials may be also include radiopaque materials, such as metallic-based powders, particulates or pastes which may be incorporated into the polymeric material. For example the radiopaque material may be blended with the polymer composition from which the polymeric wire is formed, and subsequently fashioned into the stent as described herein. Alternatively, the radiopaque material may be applied to the surface of the metal or polymer stent. In either example, various radiopaque materials and their salts and derivatives may be used including, without limitation, bismuth, barium and its salts such as barium sulphate, tantulaum, tungsten, gold, platinum and titanium, to name a few. Additional useful radiopaque materials may be found in U.S. Pat. No. 6,626,936. Metallic complexes useful as radiopaque materials are also contemplated. The stent may be selectively made radiopaque at desired areas along the wire or made be fully radiopaque, depending on the desired end-product and application. Further, the wires **38** have an inner core of tantalum, gold, platinum, iridium or combination of thereof and an outer member or layer of nitinol to provide a composite wire for improved radiocapicity or visibility. Desirably, the inner core is platinum and the outer layer is nitinol. More desirably, the inner core of platinum represents about at least 10% of the wire based on the overall cross-sectional percentage. Moreover, nitinol that has not been treated for shape memory such as by heating, shaping and cooling the nitinol at its martensitic and austenitic phases, is also useful as the outer layer. Further details of such composite wires may be found in U.S. Patent Application Publication 2002/0035396 A1.

Preferably, the wires **38** are made from nitinol, or a composite wire having a central core of platinum and an outer layer of nitinol. Further, the filling weld material, if required by welding processes such as MIG, may also be made from nitinol, stainless steel, cobalt-based alloy such as Elgiloy, platinum, gold, titanium, tantalum, niobium, and combinations thereof, preferably nitinol. The material of the cathode is no critical and can be made out of any suitable metal. The filling weld material and the wire **38** may be made of the same material, for example nitinol.

Further, the wires **38** may have a composite construction, such as described found in U.S. Patent Application Publication 2002/0035396 A1. For example, the wires **38** may have an inner core of tantalum gold, platinum, iridium or combination of thereof and an outer member or layer of nitinol to provide a composite wire for improved radiocapicity or visibility. Preferably, the wires **38** are made from nitinol.

Either or both of the opposed open ends **32, 34** of the stent **30** may have a retrieval and/or repositioning member **42** thereat. The retrieval and/or repositioning member **42** is useful for retrieval and/or repositioning of an implanted or deployed stent **30.** The retrieval and/or repositioning member **42** allows a practitioner to contract and move, reposition and/or retrieve the stent **30** within an implanted lumen (not shown). The retrieval and/or repositioning member **42** may be made from a wire, including but not limited to a memory shape alloy, such as the above described materials, including nitinol. The use of a wire, as compared other convention materials such as suture thread, has numerous advantages. For example, the self-supporting nature of the wire facilitates the locating of the retrieval and/or repositioning member. A wire will not tangle, a potential problem with suture loops, especially with suture loops made from natural or polymeric threads or filaments, and will also aid in opening the stent **30.** Another advantage from using a wire material is the wire loop defining the retrieval and/or repositioning member would be less likely to break than a plastic or polymeric loop when a pulling force is applied, such as required for repositioning or removal of the stent **30.**

As depicted in FIGS. 4-5 the stent **30** includes the retrieval and/or repositioning member **42.** The retrieval and/or repositioning member **42** includes a first section **44** and a second section **46.** The retrieval and/or repositioning member **42** may be angularly bent and extends away from the stent end **34** defining the first section **44.** The first section **44** extends upwardly away from the stent end **34** forming a loop, arc or inverted "U" shaped geometry. The retrieval and/or repositioning member **42** may include a second section **46** that extends from the angularly bent portion or either ends of the first section **44.** The second section **46** may only traverse partially about the circumference of the stent end **34.** The second section **46** may include two legs **46a, 46b** that emerge from the braid **48** of the stent **30** and cross over or under each other prior to traversing the circumference to connect to either end of the first section **44.** In other words, the legs **46a, 46b** are contained within, and a formation of, the braided pattern **48** of the stent **30.** One or more continuous wire(s) **38** is used to form stent **30.** Generally, wire **38** is bent to form the arc of the first section **44.** Wire **38** on either side of the first section **44** can be angularly bent such that the first section **44** will extend away from the stent end. Each leg **46a, 46b** of the second section **46** can extend perpendicularly from the first section **44.** Each leg **46a, 46b** can be formed into a half circle. Together the two half circles, legs **46a, 46b,** make up the second section **46** of the retrieval and/or repositioning member **42** and define the circumference of the stent end **34.** Then, the second section **46** enters into the normal braiding pattern **48** of the stent **30** and the stent body is formed using a braided pattern. The retrieval and/or repositioning member **42** may be angularly bent and extend away from the stent end **34** defining the first section **44** thereby having a length that partially circumvents the circumference of the stent end **34.** The first section **44** can be provided with a circumferential length to permit easy access by a practitioner of the retrieval and/or repositioning member **42.**

As depicted in FIG. 4, the retrieval and/or repositioning member **42** may pass through a closed-end loop **40** to attach the elongated portion or first section **44** to the stent end **32** to facilitate cinching of the stent end **32** when pulling force is applied thereto. The wire **38** forming the second section **46** of the retrieval and/or repositioning member **42** may cross through some or all of the closed-end loops **40** at stent end **32.** Some of the closed-end loops **40** may be longitudinally offset from other of the closed-end loops **40,** and the wire **38** may suitably cross through those closed-end loops **40** at the very end of the stent **30** while not crossing through the closed-end loops **40** that are disposed inwardly from the stent end **32.** Alternatively, the stent **30** may have no offsetting of the closed-end loops **40** at stent end **34.** It is desirable that the second section **46** of the retrieval and/or repositioning member **42** passes through at least two closed-end loops **40.** For example, each leg **46a, 46b** may pass through at least one closed-end loop **40** to attach each side of the elongated first section **44** to the stent end **32** to facilitate cinching of the stent end **32** when pulling force is applied thereto.

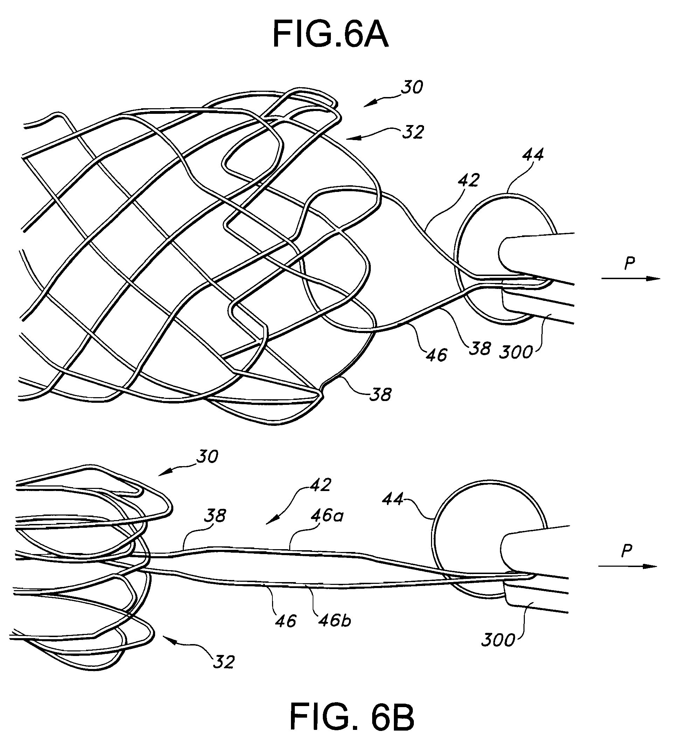

As depicted in FIGS. 6a-6b, the stent **30** should easily contract upon application of a pulling force, **"P",** to the retrieval and/or repositioning member **42.** The first section **44** of the retrieval and/or repositioning member **42** is accessed and pulled by a tool of a physician, such as by apex or loop **50** of the retrieval and/or repositioning member **42.** The legs **46a, 46b** of the second section **46** are pulled away from the stent end **34** by the pull force **(P).** The stent end **34** is axially compressed or radially contracted by a cinching action of the circumferential portion of the wire **38.** At the same time the legs **46a, 46b** are pulled away from the stent end **34,** the legs **46a, 46b** pull on the braided wire **38** extending through the braided stent body. Wire **38** is pulled toward and away from the stent end **34** causing longitudinal contraction substantially equally along the length of the stent **30.** Further, as the wire **38** forming both the first and second sections **44, 46** is integral with the braid pattern **48** of the stent **30,** such integral wire further facilitates movement, repositioning or retrieval of the stent **30** by, among other things, providing a cinching or radially contracting action substantially equally along the longitudinal length of the stent and also by transferring the pulling force along the longitudinal length of the stent. Thus, the pulling of the retrieval and/or repositioning member **42** provides for simultaneous contracting and pulling of the stent **30.** In contrast, if a pulling force **(P)** is applied to an end of a stent without having a retrieval and/or repositioning member **42,** there is no cinching or radial contracting force generated at that stent end.

As depicted in FIG. 4, the retrieval and/or repositioning member **42** desirably extends longitudinally outward from the braided stent body and about the circumference of the stent end **32** forming the second section **46** and extending outwardly away from the circumference forming a first section of the stent end **34.** Such extended and elongated retrieval and/or repositioning member **42** facilitates grabbing of the retrieval and/or repositioning member **42** by a practitioner. The first section of the retrieval and/or repositioning member may be formed in various geometric shapes to allow for ease of access by the practitioner and allow for equal longitudinal contraction of the stent. FIGS. 4-5 depict the first section **44** having a loop geometry **50.** FIGS. 8-10 depict a loop **50** bent downwardly over itself, according to the present invention. Specifically,

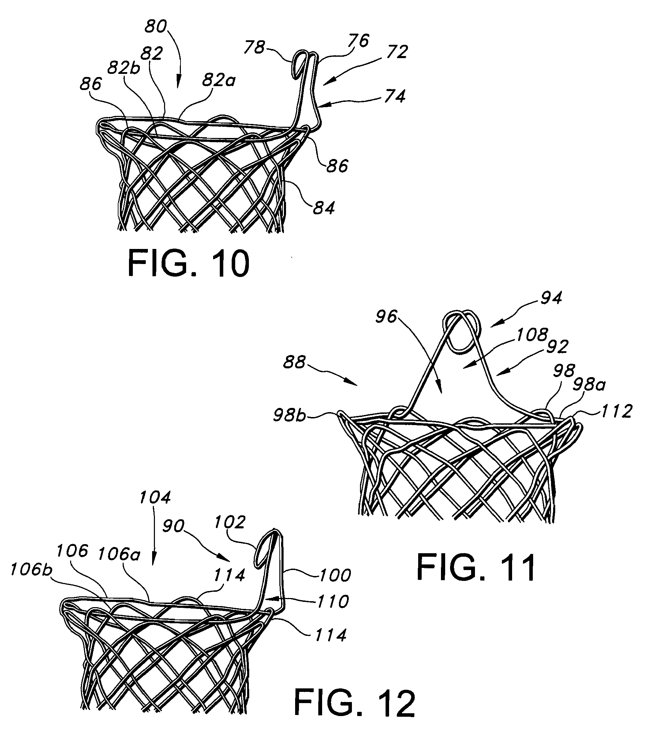

FIG. 10 depicts a first section **76** with a bent portion **78** bent downwardly over itself and bent towards the center of the stent lumen. FIGS. 8-9 depict a bent portion **56** bent downwardly towards itself and bent outwardly away from the center of the stent lumen. FIGS. 11-12 depict loops twisted into a closed-loop or pretzel shaped geometry. Specifically, FIG. 11 depicts a first section **92** twisted and bent towards the center of the stent lumen. FIG. 12 depicts a first section **100** twisted and bent outwardly away from the center of the stent lumen. The various configurations of the retrieval and/or repositioning members will be described in further detail below.

In further detail, stent **60** of FIGS. 8-9 is similar to stent **30** of FIGS. 4-5 including a braided stent body and retrieval and/or repositioning member. The retrieval and/or repositioning member **68** may include a first section **54** angularly bent and extending above the stent end; and a second section **62** extending perpendicularly from the first section and merging into and being integrally part of the formation of the braided stent body. In contrast to stent **30,** FIGS. 8-9 depict the first section **54** having a bent portion **56** instead of loop **50** of the FIGS. 4-5. The bent portion **56** forms a loop **70,** similar to loop **50,** bent downwardly toward the stent end. The bent portion **56** may be directed towards the exterior of the stent and outwardly away from the center lumen **58** of the stent **60.** FIG. 9 depicts a side view of the bent portion **56** showing the inverted "J" shape or hook shape of the bent portion **56.** FIG. 8 depicts the front view of the second section **62** showing two legs **62a, 62b** that emerge from the braided stent body **64** and cross over, or under, each other prior to traversing the circumference to connect to either end of the first section **54.** FIG. 8 shows legs **62a, 62b** interconnected or woven into a couple of closed-end loops **66.** The stent **60** advantageously permits a practitioner to grab the bent portion **56** across the hook formed by the bent portion **56**.

FIG. 10 depicts stent **72** which is similar to stent **60** of FIGS. 8-9 including a braided stent body and a retrieval and/or repositioning member. The retrieval and/or repositioning member may include a first section extending above the stent end and including a bent portion and a second section emerging from and integrally formed from the braided stent body. In contrast to stent **60,** FIG. 10 depicts the first section **76** having a bent portion **78** bent downwardly toward the stent end and inwardly toward the center lumen **80** of the stent **72.** FIG. 10 depicts a back view of the bent portion **76** showing the inverted "J" shape or hook shape of the bent shaped member. The second section **82** may include two legs **82a, 82b** that emerge from the braided stent body **84** and cross over, or under, each other prior to traversing the circumference to connect to either end of the first section **76.** Legs **82a, 82b** may be interconnected or woven into a couple of closed-end loops **76.** The stent **72** advantageously permits a practitioner to grab the bent portion **78** across the inwardly facing hook formed by the bent portion **78.**

FIGS. 11 and 12 depict stents **88** and **90,** respectively, which are similar to stents **60** and **72** of FIGS. 9 and 10, respectively, including a braided stent body and a retrieval and/or repositioning member. The retrieval and/or repositioning member includes a first section extending above the stent end and including a bent portion, and a second section emerging from and integrally formed from the braided stent body. In contrast to stents **60** and **72,** FIGS. 11 and 12 depict the first sections **92, 100,** respectively, having a bent portion which is twisted into a closed-loop, for example a pretzel shaped, geometry defining a twisted portion. FIG. 11 depicts a first section **92** including an outwardly directed twisted portion **94** which is a loop that is bent downwardly towards the stent end, directed outwardly away from the center lumen **96** and twisted in a closed-loop or pretzel formation. FIG. 11 depicts a front view of the twisted portion **94** showing the twisted inverted loop formed into a pretzel shaped geometry. The second section **98** can include two legs **98a, 98b** that emerge from the braided stent body and cross over, or under, each other prior to traversing the circumference to connect to either end of the first section **92.** Legs **98a, 98b** can be interconnected or woven into two or more closed-end loops **112.** Stent **90** of FIG. 12 is similar to stent **88** of FIG. 11. In contrast to stent **88,** stent **90** may include first section **100** including an inwardly twisted portion **102** which is a loop bent downwardly towards the stent end, directed inwardly towards the center lumen **104** and twisted in a closed-loop or pretzel formation. FIG. 12 depicts a back-side view of the twisted portion **102** showing the twisted inverted loop formed into a pretzel shaped geometry. The second section **106** includes two legs **106a, 106b** that emerge from the braided stent body and cross over, or under, each other prior to traversing the circumference to connect to either end of the first section **100.** Legs **106a, 106b** are interconnected or woven into two or more closed-end loops. Stents **88, 90** advantageously permit a practitioner to directly grab the twisted portion **94, 102** or thread a device through the triangular portions **108, 110** below the twisted portions **94**, **102**, respectively. Threading a device through the triangular portions **108, 110** will automatically thread the device through the twisted portions **94**, **102**, once a pulling force is applied to the device to remove the device from the lumen and the stent. The pretzel formation thus provides a variety of ways to latch onto the stents **88, 90.**

The retrieval and/or repositioning members of the present invention may be formed by wrapping one wire around template pins fixedly or removably disposed on a mandrel prior to winding or braiding the stent to form the first section and second section. The first section can be formed by wrapping the wire around a template pins positioned on the mandrel to cause the desired looped shape. The first section is a larger exaggerated section, such as grabbing area, for easy grabbing by the practitioner or physician. The first section may be bent or twisted as desired to form the desired geometric shape of the first section. The first section may be angularly bent and extended from the mandrel, the second section may extend from the angularly bend from the first section and perpendicularly from the first section. The second section may be formed by continuing to wrap the wire perimetrically about the mandrel forming the circumference of one end of the stent which is generally circular. A pulling force on the retrieval and/or repositioning member will cause cinching of the braid to a smaller diameter as it lengthens axially, thus allowing for less frictional force against the vessel wall and permitting retrieval and/or repositioning of the deployed stent. The retrieval and/or repositioning member wire is used to form the braided stent using the braiding technique as described herein. Additional details for braiding wires may be found in U.S. Application No. 61/147,307, filed January 26, 2009.

The retrieval and/or repositioning member can be interlaced with one or more adjacent end loops as the braided stent is being formed. Having the retrieval and/or repositioning member interlaced with one or more, and desirably at least two, adjacent closed-end loops provides for cinching of the stent end upon applying the pulling force to the retrieval and/or repositioning member.

The stent of the present invention is made from a continuous single wire strand or a multiple of single wire strands. Further, a strand may include many wires that have been welded or attached together to form the continuous single strand. For example, multiple wires may be attached end to end to form a single continuous wire without edges and free unattached ends. Once the braiding of the stent has been completed, the ends of the wire, the beginning end and the ending end, may be connected together by various means, e.g., via welding, to form a continuous closed loop braided stent. Additionally, the retrieval and/or repositioning member may also have the same or different properties than other wire(s) which form the braided stent. For example, it may be of the same or different stiffness or flexibility, all of which may be tailored for a particular application. The choice of material, wire diameter, geometry and pre-treatment of the wires and stent configuration are some of the factors which may be varied to achieve particular stent properties. Additionally, as mentioned herein, the at least one retrieval and/or repositioning member may also be made radiopaque by various methods, for example with a coating or finish, with a band or as part of the stent material, as further described herein. Color or different finishes may also be added to the retrieval and/or repositioning member to visually differentiate it from the rest of the stent wires. In some embodiments such as were polymer wires are used, attachment means may include melting the polymeric wires

The stent may have weld joints which, due to their positioning, provide higher radial strength, i.e., the resultant stents can withstand higher radial compressive forces without fear of weld failure. Wire ends to be welded may be disposed about islands or gaps on a mandrel (not shown). After the welds are formed or while the welds are being formed wire portions not forming the stent may be cut or otherwise removed from the stent braiding pattern.

Further, the stent of the present invention may have a coating. In one embodiment, the coating is a tubular covering of silicone. The stent may be placed on a coating mandrel (not shown) and may further include a tie after which the assembly can be dipped into a silicone solution to form the coating. In one embodiment, the retrieval and/or repositioning member portion is not silicone covered. In one embodiment the coating or covering may be a silicone covering, but other coverings, particularly elastomeric polymers, are useful. The coating embeds the stent therein and essentially forms a stent covering. In some embodiments when coating, it may be desirable not to embed the retrieval and/or repositioning member section in the covering, although the other wire portions emanating from the retrieval and/or repositioning member which form the braid of the stent may be coated. To prevent coating of the retrieval and/or repositioning member section, the mandrel may be truncated or geometrically altered such that it does not permit coating of the retrieval and/or repositioning member, or the retrieval and/or repositioning member can be pulled away from the mandrel during coating and formation of the polymer covering.

The stent may be fully, substantially or partially covered or lined with a polymeric material. The stent may also be embedded in a polymeric coating. The covering may be in the form of a tubular structure. Nonlimiting examples of useful polymeric materials include polyesters, polypropylenes, polyethylenes, polyurethanes, polynaphthalenes, polytetrafluoroethylenes, expanded polytetrafluoroethylene, silicone, and combinations and copolymers thereof. In some embodiments, the polymeric material is silicone. The polymeric material and/or silicone may be disposed on external surfaces of the stent, or disposed on the internal surfaces of the stent or combinations thereof.

With any embodiment, the stent may be used for a number of purposes including to maintain patency of a body lumen, vessel or conduit, such as in the coronary or peripheral vasculature, esophagus, trachea, bronchi, colon, biliary tract, pancreatic duct, urinary tract, prostate, brain, and the like. The devices of the present invention may also be used to support a weakened body lumen or to provide a fluid-tight conduit for a body lumen.

Stents of the present invention may be placed at a variety of bodily locations. In some aspects cited as examples, the tubular wall **36** of the stent **30** is disposed with a bodily lumen and one end of the stent, for example stent end **32** with the retrieval and/or repositioning member **42,** may be disposed beyond the bodily lumen being supported by the tubular wall **36** of the stent **30.** In such cases, the retrieval and/or repositioning member **42** is often disposed in a larger bodily lumen organ such that the member **42** may be more easily accessed by a practitioner. For example, the tubular wall **36** of the stent **30** may be placed within the biliary duct and the stent end **32** with the retrieval and/or repositioning member **42** may be located within the duodenum where the member **42** is more easily accessed by a practitioner. Such aspects, however, are not limiting and the stent **30** may be suitably placed with any bodily lumen and/or organ including combinations of bodily lumens and/or organs.

The stent of the present invention may be treated with a therapeutic agent or agents. "Therapeutic agents", "pharmaceuticals," "pharmaceutically active agents", "drugs" "genetic materials", "biologically active materials" and other related terms may be used interchangeably herein and include genetic therapeutic agents, non-genetic therapeutic agents and cells. The term "genetic material" means DNA or RNA, including, without limitation, DNA/RNA encoding a useful protein stated below, intended to be inserted into a human body including viral vectors and non-viral vectors. Therapeutic agents may be used singly or in combination. A wide variety of therapeutic agents can be employed in conjunction with the present invention including those used for the treatment of a wide variety of diseases and conditions (i.e., the prevention of a disease or condition, the reduction or elimination of symptoms associated with a disease or condition, or the substantial or complete elimination of a disease or condition).

The term "biological materials" include cells, yeasts, bacterial, proteins, peptides, cytokines and hormones. Examples for peptides and proteins include vascular endothelial growth factor (VEGF), transforming growth factor (TGF), fibroblast growth factor (FGF), epidermal growth factor (EGF), cartilage growth factor (CGF), nerve growth factor (NGF), keratinocyte growth factor (KGF), skeletal growth factor (SGF), osteoblast-derived growth factor (BDGF), hepatocyte growth factor (HGF), insulin-like growth factor (IGF), cytokine growth factors (CGF), platelet-derived growth factor (PDGF), hypoxia inducible factor-1 (HIF-1), stem cell derived factor (SDF), stem cell factor (SCF), endothelial cell growth supplement (ECGS), granulocyte macrophage colony stimulating factor (GM-CSF), growth differentiation factor (GDF), integrin modulating factor (IMF), calmodulin (CaM), thymidine kinase (TK), tumor necrosis factor (TNF), growth hormone (GH), bone morphogenic protein (BMP) (e.g., BMP-2, BMP-3, BMP-4, BMP-5, BMP-6 (Vgr-1), BMP-7 (PO-1), BMP-8, BMP-9, BMP-10, BMP-11, BMP-12, BMP-14, BMP-15, BMP-16, etc.), matrix metalloproteinase (MMP), tissue inhibitor of matrix metalloproteinase (TIMP), cytokines, interleukin (e.g., IL-1, IL-2, IL-3, IL-4, IL-5, IL-6, IL-7, IL-8, IL-9, IL-10, IL-11, IL-12, IL-15, etc.), lymphokines, interferon, integrin, collagen (all types), elastin, fibrillins, fibronectin, vitronectin, laminin, glycosaminoglycans, proteoglycans, transferring, cytotactin, cell binding domains (e.g., RGD), and tenascin. Exemplary BMP's are BMP-2, BMP-3, BMP-4, BMP-5, BMP-6, BMP-7. These dimeric proteins can be provided as homodimers, heterodimers, or combinations thereof, alone or together with other molecules. Cells can be of human origin (autologous or allogeneic) or from an animal source (xenogeneic), genetically engineered, if desired, to deliver proteins of interest at the transplant site. The delivery media can be formulated as needed to maintain cell function and viability. Cells include progenitor cells (e.g., endothelial progenitor cells), stem cells (e.g., mesenchymal, hematopoietic, neuronal), stromal cells, parenchymal cells, undifferentiated cells, fibroblasts, macrophage, and satellite cells.

Non-limiting examples of useful therapeutic agents include, but are not limited to, adrenergic agents, adrenocortical steroids, adrenocortical suppressants, alcohol deterrents, aldosterone antagonists, amino acids and proteins, ammonia detoxicants, anabolic agents, analeptic agents, analgesic agents, androgenic agents, anesthetic agents, anorectic compounds, anorexic agents, antagonists, anterior pituitary activators and suppressants, anthelmintic agents, anti-adrenergic agents, anti-allergic agents, anti-amebic agents, anti-androgen agents, anti-anemic agents, anti-anginal agents, anti-anxiety agents, anti-arthritic agents, anti-asthmatic agents, anti-atherosclerotic agents, antibacterial agents, anticholelithic agents, anticholelithogenic agents, anticholinergic agents, anticoagulants, anticoccidal agents, anticonvulsants, antidepressants, antidiabetic agents, antidiuretics, antidotes, antidyskinetics agents, anti-emetic agents, anti-epileptic agents, anti-estrogen agents, antifibrinolytic agents, antifungal agents, antiglaucoma agents, antihemophilic agents, antihemophilic Factor, antihemorrhagic agents, antihistaminic agents, antihyperlipidemic agents, antihyperlipoproteinemic agents, antihypertensives, antihypotensives, anti-infective agents, anti-inflammatory agents, antikeratinizing agents, antimicrobial agents, antimigraine agents, antimitotic agents, antimycotic agents, antineoplastic agents, anti-cancer supplementary potentiating agents, antineutropenic agents, antiobsessional agents, antiparasitic agents, antiparkinsonian drugs, antipneumocystic agents, antiproliferative agents, antiprostatic hypertrophy drugs, antiprotozoal agents, antipruritics, antipsoriatic agents, antipsychotics, antirheumatic agents, antischistosomal agents, antiseborrheic agents, antispasmodic agents, antithrombotic agents, antitussive agents, anti-ulcerative agents, anti-urolithic agents, antiviral agents, benign prostatic hyperplasia therapy agents, blood glucose regulators, bone resorption inhibitors, bronchodilators, carbonic anhydrase inhibitors, cardiac depressants, cardioprotectants, cardiotonic agents, cardiovascular agents, choleretic agents, cholinergic agents, cholinergic agonists, cholinesterase deactivators, coccidiostat agents, cognition adjuvants and cognition enhancers, depressants, diagnostic aids, diuretics, dopaminergic agents, ectoparasiticides, emetic agents, enzyme inhibitors, estrogens, fibrinolytic agents, free oxygen radical scavengers, gastrointestinal motility agents, glucocorticoids, gonad-stimulating principles, hemostatic agents, histamine H2 receptor antagonists, hormones, hypocholesterolemic agents, hypoglycemic agents, hypolipidemic agents, hypotensive agents, HMGCoA reductase inhibitors, immunizing agents, immunomodulators, immunoregulators, immunostimulants, immunosuppressants, impotence therapy adjuncts, keratolytic agents, LHRH agonists, luteolysin agents, mucolytics, mucosal protective agents, mydriatic agents, nasal decongestants, neuroleptic agents, neuromuscular blocking agents, neuroprotective agents, NMDA antagonists, non-hormonal sterol derivatives, oxytocic agents, plasminogen activators, platelet activating factor antagonists, platelet aggregation inhibitors, post-stroke and post-head trauma treatments, progestins, prostaglandins, prostate growth inhibitors, prothyrotropin agents, psychotropic agents, radioactive agents, repartitioning agents, scabicides, sclerosing agents, sedatives, sedative-hypnotic agents, selective adenosine A1 antagonists, adenosine A2 receptor antagonists (e.g., CGS 21680, regadenoson, UK 432097 or GW 328267), serotonin antagonists, serotonin inhibitors, serotonin receptor antagonists, steroids, stimulants, thyroid hormones, thyroid inhibitors, thyromimetic agents, tranquilizers, unstable angina agents, uricosuric agents, vasoconstrictors, vasodilators, vulnerary agents, wound healing agents, xanthine oxidase inhibitors, and the like, and combinations thereof.

Useful non-genetic therapeutic agents for use in connection with the present invention include, but are not limited to,
(a) anti-thrombotic agents such as heparin, heparin derivatives, urokinase, clopidogrel, and PPack (dextrophenylalanine proline arginine chloromethylketone);
(b) anti-inflammatory agents such as glucorticoids, betemethasone, dexamethasone, prednisolone, corticosterone, budesonide, estrogen, sulfasalazine and mesalamine;
(c) antineoplastic/ antiproliferative/anti-miotic agents such as paclitaxel, 5-fluorouracil, cisplatin, vinblastine, vincristine, epothilones, methotrexate, azathioprine, adriamycin and mutamycin, endostatin, angiostatin, angiopeptin, monoclonal antibodies capable of blocking smooth muscle cell proliferation, thymidine kinase inhibitors, cladribine, taxol and its analogs or derivatives, paclitaxel as well as its derivatives, analogs or paclitaxel bound to proteins, e.g. Abraxane™;
(d) anesthetic agents such as lidocaine, bupivacaine and ropivacaine;
(e) anti-coagulants such as D-Phe-Pro-Arg chloromethyl ketone, an RGD peptide-containing compound, heparin, hirudin, antithrombin compounds, platelet receptor antagonists, anti-thrombin antibodies, anti-platelet receptor antibodies, aspirin (aspirin is also classified as an analgesic, antipyretic and anti-inflammatory drug), dipyridamole, protamine, hirudin, prostaglandin inhibitors, platelet inhibitors, antiplatelet agents such as trapidil or liprostin and tick antiplatelet peptides;
(f) vascular cell growth promoters such as growth factors, vascular endothelial growth factors (VEGF, all types including VEGF-2), growth factor receptors, transcriptional activators, and translational promotors;
(g) vascular cell growth inhibitors such as anti-proliferative agents, growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin;
(h) protein kinase and tyrosine kinase inhibitors (e.g., tyrphostins, genistein, quinoxalines);
(i) prostacyclin analogs;
(j) cholesterol-lowering agents;
(k) angiopoietins;
(l) antimicrobial agents such as triclosan, cephalosporins, aminoglycosides and nitrofurantoin;
(m) cytotoxic agents, cytostatic agents and cell proliferation affectors;
(n) vasodilating agents;
(o) agents that interfere with endogenous vasoactive mechanisms;
(p) inhibitors of leukocyte recruitment, such as monoclonal antibodies;
(q) cytokines;
(r) hormones;
(s) inhibitors of HSP 90 protein (i.e., Heat Shock Protein, which is a molecular chaperone or housekeeping protein and is needed for the stability and function of other client proteins/signal transduction proteins responsible for growth and survival of cells) including geldanamycin;
(t) smooth muscle relaxants such as alpha receptor antagonists (e.g., doxazosin, tamsulosin, terazosin, prazosin and alfuzosin), calcium channel blockers (e.g., verapimil, diltiazem, nifedipine, nicardipine, nimodipine and bepridil), beta receptor agonists (e.g., dobutamine and salmeterol), beta receptor antagonists (e.g., atenolol, metaprolol and butoxamine), angiotensin-II receptor antagonists (e.g., losartan, valsartan, irbesartan, candesartan, eprosartan and telmisartan), and antispasmodic/anticholinergic drugs (e.g., oxybutynin chloride, flavoxate, tolterodine, hyoscyamine sulfate, diclomine);
(u) bARKct inhibitors;
(v) phospholamban inhibitors;
(w) Serca 2 gene/protein;
(x) immune response modifiers including aminoquizolines, for instance, imidazoquinolines such as resiquimod and imiquimod;
(y) human apolioproteins (e.g., AI, AII, AIII, AIV, AV, etc.);
(z) selective estrogen receptor modulators (SERMs) such as raloxifene, lasofoxifene, arzoxifene, miproxifene, ospemifene, PKS 3741, MF 101 and SR 16234;
   (aa) PPAR agonists, including PPAR-alpha, gamma and delta agonists, such as rosiglitazone, pioglitazone, netoglitazone, fenofibrate, bexaotene, metaglidasen, rivoglitazone and tesaglitazar;
   (bb) prostaglandin E agonists, including PGE2 agonists, such as alprostadil or ONO 8815Ly;
   (cc) thrombin receptor activating peptide (TRAP);
   (dd) vasopeptidase inhibitors including benazepril, fosinopril, lisinopril, quinapril, ramipril, imidapril, delapril, moexipril and spirapril;
   (ee) thymosin beta 4;
   (ff) phospholipids including phosphorylcholine, phosphatidylinositol and phosphatidylcholine;
   (gg) VLA-4 antagonists and VCAM-1 antagonists;
   (hh) anti-proliferative agents such as enoxaprin, angiopeptin, or monoclonal antibodies capable of blocking smooth muscle cell proliferation, hirudin, acetylsalicylic acid, tacrolimus, everolimus, pimecrolimus, sirolimus, zotarolimus, amlodipine and doxazosin;
   (ii) DNA demethylating drugs such as 5-azacytidine, which is also categorized as a RNA or DNA metabolite that inhibit cell growth and induce apoptosis in certain cancer cells;
   (jj) cholesterol-lowering agents, vasodilating agents, and agents which interfere with endogenous vasoactive mechanisms;
   (kk) anti-oxidants, such as probucol;
   (11) antibiotic agents, such as penicillin, cefoxitin, oxacillin, tobranycin, erythromycin, amphotericin, rapamycin (sirolimus) and adriamycin;
   (mm) angiogenic substances, such as acidic and basic fibroblast growth factors, estrogen including estradiol (E2), estriol (E3) and 17-beta estradiol;
   (nn) drugs for heart failure, such as digoxin, beta-blockers, angiotensin-convertin enzyme (ACE) inhibitors including captropril and enalopril, statins and related compounds; and
   (oo) macrolides such as sirolimus or everolimus.
The non-genetic therapeutic agents may be used individually or in combination, including in combination with any of the agents described herein.

Other therapeutic agents include nitroglycerin, nitrous oxides, nitric oxides, antibiotics, aspirins, digitalis, estrogen, estradiol, halafuginone, phospholamban inhibitors and glycosides. Exemplary therapeutic agents include anti-proliferative drugs such as steroids, vitamins, and restenosis-inhibiting agents. Exemplary restonosis-inhibiting agents include microtubule stabilizing agents such as Taxol®, paclitaxel (i.e., paclitaxel, paxlitaxel analogs, or paclitaxel derivatives, and mixtures thereof). For example, derivatives suitable for use in the medical devices include 2'-succinyl-taxol, 2'-succinyl-taxol triethanolamine, 2'-glutaryl-taxol, 2'glutaryl-taxol triethanolamine salt, 2'-O-ester with N-(dimethylaminoethyl)glutamine, and 2'-O-ester with N-(dimethylaminoethyl)glutamide hydrochloride salt.

Further examples of non-genetic therapeutic agents, not necessarily exclusive of those listed above, include taxanes such as paclitaxel (including particulate forms thereof, for instance, protein-bound paclitaxel particles such as albumin-bound paclitaxel nanoparticles, e.g., Abraxane™), sirolimus, everolimus, tacrolimus, zotarolimus, Epo D, dexamethasone, estradiol, halofuginone, cilostazole, geldanamycin, alagebrium chloride (ALT-711), ABT-578 (Abbott Laboratories), trapidil, liprostin, Actinomcin D, Resten-NG, Ap-17, abciximab, clopidogrel, Ridogrel, beta-blockers, bARKct inhibitors, phospholamban inhibitors, Serca 2 gene/protein, imiquimod, human apolioproteins (e.g., AI-AV), growth factors (e.g., VEGF-2) , as well derivatives of the forgoing, among others.

Useful genetic therapeutic agents for use in connection with the present invention include, but are not limited to, anti-sense DNA and RNA as well as DNA coding for the various proteins (as well as the proteins themselves), such as (a) anti-sense RNA; (b) tRNA or rRNA to replace defective or deficient endogenous molecules; (c) angiogenic and other factors including growth factors such as acidic and basic fibroblast growth factors, vascular endothelial growth factor, endothelial mitogenic growth factors, epidermal growth factor, transforming growth factor α and β, platelet-derived endothelial growth factor, platelet-derived growth factor, tumor necrosis factor α, hepatocyte growth factor and insulin-like growth factor; (d) cell cycle inhibitors including CD inhibitors, and (e) thymidine kinase ("TK") and other agents useful for interfering with cell proliferation. DNA encoding for the family of bone morphogenic proteins ("BMP's") are also useful and include, but not limited to, BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently desirably BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors for delivery of genetic therapeutic agents include, but not limited to, viral vectors such as adenoviruses, gutted adenoviruses, adeno-associated virus, retroviruses, alpha virus (Semliki Forest, Sindbis, etc.), lentiviruses, herpes simplex virus, replication competent viruses (e.g., ONYX-015) and hybrid vectors; and non-viral vectors such as artificial chromosomes and mini-chromosomes, plasmid DNA vectors (e.g., pCOR), cationic polymers (e.g., polyethyleneimine, polyethyleneimine (PEI)), graft copolymers (e.g., polyether-PEI and polyethylene oxide-PEI), neutral polymers such as polyvinylpyrrolidone (PVP), SP1017 (SUPRATEK), lipids such as cationic lipids, liposomes, lipoplexes, nanoparticles, or microparticles, with and without targeting sequences such as the protein transduction domain (PTD).

Cells for use in connection with the present invention may include cells of human origin (autologous or allogeneic), including whole bone marrow, bone marrow derived mononuclear cells, progenitor cells (e.g., endothelial progenitor cells), stem cells (e.g., mesenchymal, hematopoietic, neuronal), pluripotent stem cells, fibroblasts, myoblasts, satellite cells, pericytes, cardiomyocytes, skeletal myocytes or macrophage, or from an animal, bacterial or fungal source (xenogeneic), which can be genetically engineered, if desired, to deliver proteins of interest.

Numerous therapeutic agents, not necessarily exclusive of those listed above, have been identified as candidates for vascular treatment regimens, for example, as agents targeting restenosis (antirestenotics). Such agents are useful for the practice of the present invention and include one or more of the following:
(a) Ca-channel blockers including benzothiazapines such as diltiazem and clentiazem, dihydropyridines such as nifedipine, amlodipine and nicardapine, and phenylalkylamines such as verapamil;
(b) serotonin pathway modulators including: 5-HT antagonists such as ketanserin and naftidrofuryl, as well as 5-HT uptake inhibitors such as fluoxetine;
(c) cyclic nucleotide pathway agents including phosphodiesterase inhibitors such as cilostazole and dipyridamole, adenylate/Guanylate cyclase stimulants such as forskolin, as well as adenosine analogs;
(d) catecholamine modulators including α-antagonists such as prazosin and bunazosine, β-antagonists such as propranolol and α/β-antagonists such as labetalol and carvedilol;
(e) endothelin receptor antagonists such as bosentan, sitaxsentan sodium, atrasentan, endonentan;
(f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine;
(g) Angiotensin Converting Enzyme (ACE) inhibitors such as cilazapril, fosinopril and enalapril;
(h) ATII-receptor antagonists such as saralasin and losartin;
(i) platelet adhesion inhibitors such as albumin and polyethylene oxide;
(j) platelet aggregation inhibitors including cilostazole, aspirin and thienopyridine (ticlopidine, clopidogrel) and GP IIb/IIIa inhibitors such as abciximab, epitifibatide and tirofiban;
(k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C;
(l) cyclooxygenase pathway inhibitors such as aspirin, ibuprofen, flurbiprofen, indomethacin and sulfinpyrazone;
(m) natural and synthetic corticosteroids such as dexamethasone, prednisolone, methprednisolone and hydrocortisone;
(n) lipoxygenase pathway inhibitors such as nordihydroguairetic acid and caffeic acid;
(o) leukotriene receptor antagonists; (p) antagonists of E- and P-selectins;
(q) inhibitors of VCAM-1 and ICAM-1 interactions;
(r) prostaglandins and analogs thereof including prostaglandins such as PGE1 and PGI2 and prostacyclin analogs such as ciprostene, epoprostenol, carbacyclin, iloprost and beraprost;
(s) macrophage activation preventers including bisphosphonates;
(t) HMG-CoA reductase inhibitors such as lovastatin, pravastatin, atorvastatin, fluvastatin, simvastatin and cerivastatin;
(u) fish oils and omega-3-fatty acids;
(v) free-radical scavengers/antioxidants such as probucol, vitamins C and E, ebselen, trans-retinoic acid, SOD (orgotein) and SOD mimics, verteporfin, rostaporfin, AGI 1067, and M 40419;
(w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-β pathway agents such as polyanionic agents (heparin, fucoidin), decorin, and TGF-β antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives;
(x) matrix metalloprotease (MMP) pathway inhibitors such as marimastat, ilomastat, metastat, batimastat, pentosan polysulfate, rebimastat, incyclinide, apratastat, PG 116800, RO 1130830 or ABT 518;
(y) cell motility inhibitors such as cytochalasin B;
(z) antiproliferative/antineoplastic agents including antimetabolites such as purine antagonists/analogs (e.g., 6-mercaptopurine and pro-drugs of 6-mercaptopurine such as azathioprine or cladribine, which is a chlorinated purine nucleoside analog), pyrimidine analogs (e.g., cytarabine and 5-fluorouracil) and methotrexate, nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, Epo D, paclitaxel and epothilone), caspase activators, proteasome inhibitors, angiogenesis inhibitors (e.g., endostatin, angiostatin and squalamine), olimus family drugs (e.g., sirolimus, everolimus, tacrolimus, zotarolimus, etc.), cerivastatin, flavopiridol and suramin;
   (aa) matrix deposition/organization pathway inhibitors such as halofuginone or other quinazolinone derivatives, pirfenidone and tranilast;
   (bb) endothelialization facilitators such as VEGF and RGD peptide;
   (cc) blood rheology modulators such as pentoxifylline and
   (dd) glucose cross-link breakers such as alagebrium chloride (ALT-711).
These therapeutic agents may be used individually or in combination, including in combination with any of the agents described herein.

Numerous additional therapeutic agents useful for the practice of the present invention are also disclosed in U.S. Patent No. 5,733,925 to Kunz.

A wide range of therapeutic agent loadings may used in connection with the dosage forms of the present invention, with the pharmaceutically effective amount being readily determined by those of ordinary skill in the art and ultimately depending, for example, upon the condition to be treated, the nature of the therapeutic agent itself, the tissue into which the dosage form is introduced, and so forth.

Further, with any embodiment of the stent the general tubular shape may be varied. For example, the tubular shape may have a varied diameter, may be tapered, and may have an outwardly flared end and the like. Further, the ends of the stent may have a larger diameter than the middle regions of the stent. In one particularly useful embodiment, at least one of the ends of the stent transition from one diameter to another diameter. Desirably, both ends transition in this manner to yield "flared" ends.

The stent may be coated with a polymeric material. For example, the stent wires may be partially or fully covered with a biologically active material which is elutably disposed with the polymeric material. Further, the polymeric coating may extend over or through the interstitial spaces between the stent wires so as to provide a hollow tubular liner or cover over the interior or the exterior surface of the stent. The polymeric material may be selected from the group consisting of polyester, polypropylene, polyethylene, polyurethane, polynaphthalene, polytetrafluoroethylene, expanded polytetrafluoroethylene, silicone, and combinations thereof.

Various stent types and stent constructions may be employed in the invention. Among the various stents useful include, without limitation, self-expanding stents and balloon expandable extents. The stents may be capable of radially contracting, as well and in this sense can best be described as radially distensible or deformable. Self-expanding stents include those that have a spring-like action which causes the stent to radially expand, or stents which expand due to the memory properties of the stent material for a particular configuration at a certain temperature. Nitinol is one material which has the ability to perform well while both in spring-like mode, as well as in a memory mode based on temperature. Other materials are of course contemplated, such as stainless steel, platinum, gold, titanium and other biocompatible metals, as well as polymeric stents. The configuration of the stent may also be chosen from a host of geometries, as long as said geometries fall within the scope of the appended claims. For example, wire stents can be fastened into a continuous helical pattern, with or without a wave-like or zig-zag in the wire, to form a radially deformable stent. Individual rings or circular members can be linked together such as by struts, sutures, welding or interlacing or locking of the rings to form a tubular stent. Tubular stents also include those formed by etching or cutting a pattern from a tube. Such stents are often referred to as slotted stents. Furthermore, stents may be formed by etching a pattern into a material or mold and depositing stent material in the pattern, such as by chemical vapor deposition or the like. Examples of various stent configurations are shown in U.S. Pat. No. 4,503,569 to Dotter; U.S. Pat. No. 4,733,665 to Palmaz; U.S. Pat. No. 4,856,561 to Hillstead; U.S. Pat. No. 4,580,568 to Gianturco; U.S. Pat. No. 4,732,152 to Wallsten, U.S. Pat. No. 4,886,062 to Wiktor, and U.S. Pat. No. 5,876,448 to Thompson, U.S. Pat. Nos. 6,007,574, 6,309,415, 7,60,323, 7,419,502 and 7,419,503 to Pulnev et el.; U.S. Pat. No. 7,311,031 to McCullagh et al.; and U.S. Pat. Application Publication No. 2007/0118206 to Colgan et al.,.

The invention being thus described, it will now be evident to those skilled in the art that the same may be varied in many ways. Such variations are not to be regarded as a departure from the scope of the invention as long as they fall within the scope of the following claims. Further, any of the embodiments or aspects of the invention as described in the claims may be used with one and another without limitation.

### FURTHER DEVICES ARE GIVEN BELOW, AS EXAMPLES NOT FORMING PART OF THE INVENTION.

1. An implantable device comprising:
   one or more elongated wires braided to form a tubular device having opposed first open end, a second open end, a tubular body therebetween defining a device lumen therethrough with an interior surface and an exterior surface, and a retrieval and/or
   repositioning member integrally formed from a single of said elongated wires, said retrieval and/or repositioning member includes an elongated portion extending from said first open end and said retrieval and/or repositioning member interlooping circumferentially about said first open end whereby force exerted on said elongated portion causes radially contraction of said tubular stent and cinching of said first open end.
2. The device of paragraph 1, wherein said first open end is defined by a series of closed-end loops and said retrieval and/or repositioning member passes through at least two closed-end loops attaching said elongated portion to said first open end.
3. The device of paragraph 1, wherein said retrieval and/or repositioning member consists essentially of said single wire which extends about said first open end and crosses over itself prior to being incorporated into said tubular body.
4. The device of paragraph 1, wherein said elongated portion of said retrieval and/or repositioning member is a single wire loop.
5. The device of paragraph 4, wherein said single wire loop is bent inwardly towards said stent lumen forming an inwardly directed bent portion.
6. The device of paragraph 4, wherein said single wire loop is bent outwardly away from said device lumen forming an externally directed bent portion.
7. The device of paragraph 4, wherein said inwardly directed bent portion is twisted into a knot.
8. The device of paragraph 1, wherein said retrieval and/or repositioning member includes two elongated portions extending from said first open end forming two single wire loops.
9. The device of paragraph 8, wherein each of said two elongated portions are loops bent into a hook shaped geometry directed outwardly away from said device lumen.
10. The device of paragraph 8, wherein each of said two elongated portions are loops bent into a hook shaped geometry directed inwardly toward said device lumen.
11. The device of paragraph 1, wherein said wire comprises metallic and/or polymeric materials.
12. The device of paragraph 1, further comprising a covering disposed over at least a portion of a device surface.
13. A stent comprising:
   one or more elongated wires braided to form a tubular stent having a retrieval and/or repositioning member and opposed first open end and a second open end with each open end having a circumference and a tubular body therebetween, said first open end is defined by series of closed-end loops, said retrieval and/or repositioning member having a first section including at least one elongated closed-end loop extending from said first open end and a second section emerging from said braided tubular body, interwoven with at least one closed- end loop and integrally extending into said first section whereby force exerted on said elongated closed-end loop causes radially contraction of said tubular stent.
14. A method for producing a tubular braided stent having opposed first stent end and second stent end and having an integral retrieval and/or repositioning loop at the first stent end, comprising:
   selecting one or more elongate wires having opposed ends;
   forming a retrieval and/or repositioning member from single of said wires comprising an elongated loop which extends above and beyond said first stent end to permit grabbing of said loop by a practitioner to radially contract said stent; and
   braiding said one or more wires to form said stent.
15. The method of paragraph 14, wherein the step for braiding includes interweaving said retrieval and/or repositioning loop circumferentially into said first stent end.
16. The method of paragraph 15, further comprising bending said elongated loop to form a bent hook portion extending outwardly from said stent.
17. The method of paragraph 15, further comprising bending said elongated loop to form a bent hook portion extending inwardly towards said stent end.
18. The method of paragraph 17, further comprising twisting said bent hook portion to form a twisted knot shaped portion.
19. The method of paragraph 14, wherein said step of forming a retrieval and/or repositioning member includes forming two opposing elongated loops which extend above and beyond said first stent end to permit grabbing of said loops by a practitioner to radially contract said stent.
20. A delivery system comprising:
   a delivery catheter; and
   a stent comprising: one or more elongated wires braided to form a tubular stent having opposed first open end, a second open end, a tubular body therebetween defining a stent lumen therethrough with an interior surface and an exterior surface, and a retrieval and/or repositioning member integrally formed from a single of said elongated wires, said retrieval and/or repositioning member includes an elongated portion extending from said first open end and said retrieval and/or repositioning member interwoven circumferentially about said first open end whereby force exerted on said elongated portion causes radially contraction of said tubular stent and cinching of said first open end.

## Claims

1. An implantable stent comprising:
one or more elongated wires (14, 18, 38) braided to form a tubular stent having opposed first open end (32), a second open end (34), a tubular body therebetween defining a stent lumen therethrough with an interior surface and an exterior surface, and a retrieval and/or repositioning member integrally formed from a single of said elongated wires (14, 18, 38), said retrieval and/or repositioning member (42, 68) including an elongated portion extending from said first open end (32) and said retrieval and/or repositioning member (42, 68) interlooping circumferentially about said first open end (32) whereby force exerted on said elongated portion causes radial contraction of said tubular stent and cinching of said first open end (32), **characterized in that** said elongated portion of said retrieval and/or repositioning member (42, 68) is a single wire loop bent downwardly toward the stent end (32) into a hook shaped geometry.

2. The stent of claim 1, wherein said first open end (32) is defined by a series of closed-end loops (40, 76) and said retrieval and/or repositioning member (42, 68) passes through at least two closed-end loops (40, 76) attaching said elongated portion to said first open end.

3. The stent of claim 1, wherein said retrieval and/or repositioning member (42, 68) consists essentially of said single wire which extends about said first open end (32) and crosses over itself prior to being incorporated into said tubular body (36).

4. The stent of claim 1, wherein said single wire loop is bent inwardly towards said stent lumen forming an inwardly directed bent portion.

5. The stent of claim 1, wherein said single wire loop is bent outwardly away from said stent lumen forming an externally directed bent portion.

6. The stent of claim 4, wherein said inwardly directed bent portion is twisted into a knot.

7. The stent of claim 1, wherein said retrieval and/or repositioning member (42, 68) includes two elongated portions extending from said first open end (32) forming two single wire loops.

8. The stent of claim 7, wherein each of said two elongated portions are loops bent into a hook shaped geometry directed outwardly away from said stent lumen.

9. The stent of claim 7, wherein each of said two elongated portions are loops bent into a hook shaped geometry directed inwardly toward said stent lumen.

10. The stent of claim 1, wherein said wire comprises metallic and/or polymeric materials.

11. The stent of claim 1, further comprising a covering disposed over at least a portion of a stent surface.

## Patentansprüche

1. Implantierbarer Stent, der aufweist:
einen oder mehrere längliche Drähte (14, 18, 38), die so geflochten sind, dass sie einen röhrenförmigen Stent mit einem ersten offenen Ende (32), einem entgegengesetzten zweiten offenen Ende (34), einem röhrenförmigen Körper dazwischen, der ein durchgehendes Stentlumen mit einer Innenfläche und einer Außenfläche definiert, und ein Bergungs- und/oder Repositionierungsteil, das aus einem einzelnen der länglichen Drähte (14, 18, 38) in einem Stück gebildet ist, wobei das Bergungs- und/oder Repositionierungsteil (42, 68) einen länglichen Abschnitt aufweist, der sich vom ersten offenen Ende (32) erstreckt, und das Bergungs- und/oder Repositionierungsteil (42, 68) um das erste offene Ende (32) über den Umfang vermascht ist, wodurch auf den länglichen Abschnitt ausgeübte Kraft Radialkontraktion des röhrenförmigen Stents und Zusammenziehen des ersten offenen Endes (32) bewirkt, **dadurch gekennzeichnet, dass** der längliche Abschnitt des Bergungs- und/oder Repositionierungsteils (42, 68) eine einzelne Drahtschlaufe ist, die nach unten zum Stentende (32) in eine hakenförmige Geometrie gebogen ist.

2. Stent nach Anspruch 1, wobei das erste offene Ende (32) durch eine Folge geschlossener Endschlaufen (40, 76) definiert ist und das Bergungs- und/oder Repositionierungsteil (42, 68) mindestens zwei geschlossene Endschlaufen (40, 76) durchläuft, was den länglichen Abschnitt am ersten offenen Ende befestigt.

3. Stent nach Anspruch 1, wobei das Bergungs- und/oder Repositionierungsteil (42, 68) im Wesentlichen aus dem einzelnen Draht besteht, der sich um das erste offene Ende (32) erstreckt und sich selbst überkreuzt, bevor er in den röhrenförmigen Körper (36) eingearbeitet ist.

4. Stent nach Anspruch 1, wobei die einzelne Drahtschlaufe nach innen zum Stentlumen gebogen ist, was einen nach innen gerichteten gebogenen Abschnitt bildet.

5. Stent nach Anspruch 1, wobei die einzelne Drahtschlaufe nach außen vom Stentlumen weg gebogen ist, was einen nach außen gerichteten gebogenen Abschnitt bildet.

6. Stent nach Anspruch 4, wobei der nach innen gerichtete gebogene Abschnitt zu einem Knoten verdrillt ist.

7. Stent nach Anspruch 1, wobei das Bergungs- und/oder Repositionierungsteil (42, 68) zwei längliche Abschnitte aufweisen, die sich vom ersten offenen Ende (32) erstrecken, was zwei einzelne Drahtschlaufen bildet.

8. Stent nach Anspruch 7, wobei jeder der beiden länglichen Abschnitte eine Schlaufe ist, die in eine hakenförmige Geometrie gebogen ist, die vom Stentlumen weg nach außen gerichtet ist.

9. Stent nach Anspruch 7, wobei jeder der beiden länglichen Abschnitte eine Schlaufe ist, die in eine hakenförmige Geometrie gebogen ist, die zum Stentlumen nach innen gerichtet ist.

10. Stent nach Anspruch 1, wobei der Draht Metall- und/oder Polymermaterialien aufweist.

11. Stent nach Anspruch 1, der ferner eine Abdeckung aufweist, die über mindestens einem Abschnitt einer Stentoberfläche angeordnet ist.

## Revendications

1. Stent implantable comprenant :
un ou plusieurs fils allongés (14, 18, 38) tressés pour former un stent tubulaire ayant une première extrémité ouverte (32) opposée, une seconde extrémité ouverte (34), un corps tubulaire entre elles définissant une lumière de stent à travers ces dernières avec une surface intérieure et une surface extérieure, et un élément de récupération et/ou de repositionnement formé de manière solidaire à partir d'un seul desdits fils allongés (14, 18, 38), ledit élément de récupération et/ou de repositionnement (42, 68) comprenant une partie allongée s'étendant à partir de ladite première extrémité ouverte (32) et ledit dispositif de récupération et/ou repositionnement (42, 68) formant des boucles de manière circonférentielle autour de ladite première extrémité ouverte (32), moyennant quoi la force exercée sur ladite partie allongée provoque la contraction radiale dudit stent tubulaire et la fixation de ladite première extrémité ouverte (32), **caractérisé en ce que** ladite partie allongée dudit élément de récupération et/ou de repositionnement (42, 68) est une simple boucle de fil pliée vers le bas vers l'extrémité de stent (32) dans une géométrie en forme de crochet.

2. Stent selon la revendication 1, dans lequel ladite première extrémité ouverte (32) est définie par une série de boucles à extrémité fermée (40, 76) et ledit élément de récupération et/ou de repositionnement (42, 68) passe à travers au moins deux boucles à extrémité fermée (40, 76) fixant ladite partie allongée à ladite première extrémité ouverte.

3. Stent selon la revendication 1, dans lequel ledit élément de récupération et/ou de repositionnement (42, 68) se compose essentiellement dudit fil unique qui s'étend autour de ladite première extrémité ouverte (32) et se croise sur lui-même avant d'être incorporé dans ledit corps tubulaire (36).

4. Stent selon la revendication 1, dans lequel ladite boucle de fil unique est pliée vers l'intérieur, vers ladite lumière de stent formant une partie pliée dirigée vers l'intérieur.

5. Stent selon la revendication 1, dans lequel ladite boucle de fil unique est pliée vers l'extérieur, à distance de ladite lumière de stent formant une partie pliée dirigée vers l'extérieur.

6. Stent selon la revendication 4, dans lequel ladite partie pliée dirigée vers l'intérieur est torsadée en un noeud.

7. Stent selon la revendication 1, dans lequel ledit élément de récupération et/ou de repositionnement (42, 68) comprend deux parties allongées s'étendant à partir de ladite première extrémité ouverte (32) formant deux boucles de fil uniques.

8. Stent selon la revendication 7, dans lequel chacune desdites deux parties allongées sont des boucles pliées en une géométrie en forme de crochet dirigées vers l'intérieur à distance de ladite lumière de stent.

9. Stent selon la revendication 7, dans lequel chacune desdites deux parties allongées est une boucle pliée selon une géométrie en forme de crochet dirigée vers l'extérieur à distance de ladite lumière de stent.

10. Stent selon la revendication 1, dans lequel ledit fil comprend des matériaux métalliques et/ou polymères.

11. Stent selon la revendication 1, comprenant en outre un revêtement disposé sur au moins une partie d'une surface de stent.
